# EUROPÄISCHE PATENTSCHRIFT

(11) **EP 2 552 372 B1**
(45) Veröffentlichungstag und Bekanntmachung des Hinweises auf die Patenterteilung: **12.05.2021**
(21) Anmeldenummer: 11751794.6
(22) Anmeldetag: 27.03.2011
(51) Int. Cl.: A61F 13/15, A61F 13/532, A61F 13/539, A61F 13/49, B32B 5/02, B32B 5/04, B32B 5/26, B32B 7/12, A61F 13/53, A61F 13/534

(54) **VERFAHREN ZUR HERSTELLUNG EINES HOCHFLEXIBLEN ABSORBIERENDEN LAMINATS**
METHOD FOR THE PRODUCTION OF HIGHLY FLEXIBLE ABSORBENT LAMINATE
PROCÉDÉ DE FABRICATION D'UN PRODUIT STRATIFIÉ ABSORBANT TRÈS FLEXIBLE

(30) Priorität: 29.03.2010 DE 102010013288
(43) Veröffentlichungstag der Anmeldung: 06.02.2013
(73) Patentinhaber: Evonik Operations GmbH, 45128 Essen (DE)
(72) Erfinder: FENSKE, Wilfried, 64 683 Einhausen (DE)
(74) Vertreter: Evonik Patent Association
(86) Internationale Anmeldenummer: PCT/DE2011/000339
(87) Internationale Veröffentlichungsnummer: WO 2011/120504

(56) Entgegenhaltungen:
- WO-A1-2004/011046
- JP-A- 2002 192 641
- US-A- 5 366 452
- US-A1- 2006 206 073
- US-A1- 2008 156 418

## Beschreibung

Textile, absorbierende Materialien, welche zumindesten in einer Koordinatenrichtung ausdehnbar sind und der Volumenzunahme durch Aufnahme der zu absorbierenden Flüssigkeit Raum schaffen, werden insbesondere im Zusammenhang mit Einwegartikeln der Babywindeln, Inkontinenzprodukte und Damenhygiene, sowie im Bereich absorbierender Artikel der Verpackungs- und Lebensmitteltechnik nachgefragt. Traditionell wird diese Aufgabe durch geeignete Kombinationsprodukte aus Zellstoff, superabsorbierende Granulate oder -fasern und flüssigkeitsverteilenden Schichten aus synthetischen Vliesstoffen, Zellstoff oder Baumwolle erfüllt.

In Erkenntnis der Verantwortung zur Nachhaltigkeit der Schätze dieser Erde und dem bewußten Umgang mit den Folgen ihrer Nutzung ist es die Verpflichtung der Technik, den Verbrauch an Rohstoffen und Verpackungsmaterialien und den Transportenergien für Produktion und Vertrieb so gering wie möglich zu halten. Für die Herstellung von Hygieneartikeln bedeutet dies in erster Linie die Reduzierung von Primärenergieverbrauch, Zellstoff, Transportvolumen, Verpackungsfolien und -kartonage und Abfallvolumen.

Speziell im Bereich Babywindeln sind sowohl aus der Literatur, als auch aus der industriellen Ausführung vielfältige Projekte bekannt, die den teilweisen oder vollständigen Ersatz der Saugfähigkeit von Zellulose-Zellstoff durch superabsorbierende Granulate (Superabsorber) anstreben und auch erreichen. So ist es technisch, ökonomisch und ökologisch wünschenswert, beispielsweise moderne Babywindeln oder Inkontinenzartikel für Erwachsene komplett auf zellstofffreie Saugkissen umzustellen, um den Einfluß der Verwendung von Einwegartikeln auf die Klimaentwicklung so gering wie möglich zu halten.

### STAND DER TECHNIK

Es sind zwei grundsätzlich unterschiedliche Ansätze zur Reduzierung, bzw Eliminierung des Zellstoffs aus den absorbierenden Kissen von Babywindeln, Inkontinenzprodukten und Damenhygieneartikeln bekannt:
1. Ersatz des Zellstoffs durch dehn- oder quellfähige Thermoplaste als Binder zwischen den Granulaten des Superabsorbers, welche beim Aufquellen des Superabsorbers die Haftung erhalten, die Quellbewegung mitmachen und die Integrität des Substrats auch in feuchtem Zustand gewährleisten.
2. Einlagerung des Superabsorbers zwischen zwei oder mehrerer Trägerschichten in diskreten Sektionen im Sinne einer Steppdecke, wobei die Volumenvergrößerung des Superabsorbers durch dessen Quellung durch Flüssigkeitsaufnahme alternativ durch Elastizität von einer oder mehrerer Trägerschichten, Raffung oder Dehnung einer oder mehrerer Trägerschichten während der Einbringung des Superabsorbers in das Laminat oder durch geeignete Verbindung der einzelnen Trägerschichten zueinander derart, daß ein gezieltes, lokales Aufplatzen dieser Verbindung aufgrund des Berstdrucks durch die Quellung des Superabsorbers ermöglicht wird.

In EP 724418 wird die Herstellung eines Laminats beschrieben, bestehend aus zwei äußeren Lagen, von welchen wenigstens eine Lage hydrophil ist und welche derart mittels eines wassersensiblen Haftklebers zueinander verleimt sind, daß in isolierten unverleimten Sektionen punktuell Superabsorber eingelagert werden kann, welches im gequollenen Zustand gezielt die Verleimung aufbricht und die für das Quellvolumen erforderliche Volumenerhöhung des Laminats erreicht. Es ist anzunehmen, daß im gequollenem Zustand nur eine begrenzte Integrität des Laminats erreicht werden kann.

Ein Verfahren zur Herstellung eines saugfähigen Laminats mit eingelagerten Sektionen von Superabsorber und elastischen Eigenschaften ist in US 20020102392 vorgestellt. Zwischen zwei äußere Lagen, von denen eine in Längsrichtung mittels einer Profilwalze gerafft wird, werden quer zur Produktionsrichtung Sektionen von Superabsorbern über ein Vakuumsystem positioniert und ein in Längsrichtung dehnbares Laminat erzielt, wobei die Elastizität und Raffung duch Verwendung elastischer Folien oder Vliesstoffe erhöht werden kann.

Ein Verfahren zur längskontinuierlichen Herstellung eines Laminats mit Einzelbahnen von Superabsorber, die mittels Heißleims zwischen zwei äußere Lagen dergestalt eingebracht sind, daß sich quer zur Produktionsrichtung jeweils Streifen von superabsorberbedeckten und superabsorberfreien Bereichen ausbilden, ist in US20020115969 ausgeführt.

Die Herstellung eines Laminats aus zwei äußeren Lagen mit punktuell eingelagerten Sektionen von Superabsorbern wird in WO 2004071539 und WO 2004071363 beschrieben. Mittels einer texturierten Vakuumwalze wird eine erste äußere Schicht zur Ausbildung von Vertiefungen gebracht, diese mit Superabsorbern und Faserstoffen gefüllt und mit der zweiten äußeren Schicht verbunden. Vergleichbare Produkte werden seit längerem auch als Unterlagen im OP-Bereich und in der Krankenversorgung verwendet.

US 2008/156418 A1 offenbart ein biegeweiches, flüssigkeitsabsorbierendes Bahnmaterial mit zwei äußeren Schichten, von denen wenigstens eine von einem textilen Material gebildet wird, zwischen denen mindestens zwei gegenüber diesen Schichten unter Vorspannung stehende dehnelastische Zwischenlagen aus schräg zur Produktionsrichtung gegenläufig angeordnete Fäden oder Bändern eingebracht sind, durch welche das Bahnmaterial im Wesentlichen quer zu seiner Herstellungsrichtung unter Entspannung der Zwischenlage verkürzt und gerafft wird und bei dem in den Freiräumen der elastischen Zwischenlage pulver- oder faserförmige Einlagerungen eingebracht sind, welche absorbierende Eigenschaften gegenüber Flüssigkeiten wie etwa Urin, Blut, Wasser oder Schweiß aufweisen.

Die Herstellung des aus US 2008/156418 A1 bekannten, biegeweichen Bahnmaterial erfolgt derart, dass eine textile äußere Schicht über einen langgestreckten Formkern zu einem Schlauch gefaltet und mit Gruppen elastischer Fäden oder Bänder gegenläufig im Sinne einer Schlaucharmierung umwickelt wird, auf diese Gruppen elastischer Elemente Haftkleber aufgebracht und diese über den Formkern unter Spannung mit dem Schlauch der ersten äußeren Schicht in Kontakt kommen, beide zusammen im weiteren Verlauf der Vorschubbewegung des Schlauches auf den Formkern und unter intermittierender oder kontinuierlicher Zufuhr von Einzelspuren von Superabsorbern mit einer zweiten äußeren Schicht verbinden und längs zu querelastischen Einzelbahnen aufgeschnitten wird.

Sowohl die US 5 366 452 A als auch die WO 2004/011046 A1 offenbaren ein elastisches, biegeweiches und absorbierendes oder resorbierendes Bahnmaterial.

US 2006/206073 A1 offenbart einen Absorptionskern für ein Hygieneprodukt welches eine textile Lagen umfasst, die ein Berg und ein Tal ausbildet. In dem Tal ist Superabsorber angeordnet.

JP 2002 192641 A beschreibt ein Verfahren zur Herstellung eines in Querrichtung dehn- bzw. schrumpfbares Verbundmaterial.

### BESCHREIBUNG DER ERFINDUNG

Superabsorber erfahren während der Flüssigkeitsaufnahme eine Gewichtszunahme von 2500-5000%. Die damit einhergehende Volumenvergrößerung gilt es, durch geeignete Flexibilität des umgebenden Trägermaterials aufzunehmen. Im Falle klassischer Zellstoff/Superabsorber Saugkissen ist dies grundsätzlich kein Problem, da der Zellstoff Ausdehnung in allen drei Dimensionen ermöglicht. Im Falle sogenannter Superabsorber-Laminate, in denen Superabsorber-Granulat oder Superabsorber/Fasermischungen mittels Haftkleber oder thermisch zwischen zwei oder mehrere Lagen Nonwovens, Folie, Tissue, o.ä. fixiert wird, müssen die äußeren Lagen des Laminats diese Funktion ermöglichen, sei es durch Dehnung oder durch geometrische Flexibilität. Dabei muß hingegen die Einschließung des Superabsorbers jederzeit gewährleistet sein, ohne daß es zum Bruch einer der äußeren Lagen oder zum Aufreißen der Laminierung kommt. Es ist weiterhin wünschenswert, daß dieses Laminat nicht nur senkrecht zu seiner Herstellungsebene, sondern auch in dieser Ebene selbst elastisch dehnbar ist, um sowohl die Volumenvergrößerung des Superabsorbers durch die Flüssigkeitsaufnahme zu erleichtern, als auch in Kombination mit anderen Komponenten der oben bezeichneten Hygieneartikeln deren Flexibilität und Anpassungsfähigkeit an die jeweilige Körperkontur nicht zu beeinträchtigen.

Der Erfindung liegt damit die Aufgabe zugrunde, ein Herstellungsverfahren zu schaffen, welches die kontinuierliche Herstellung von saugfähigem textilen Bahnmaterial mit hoher Produktionskapazität -hieraus resultierend- preisgünstig ermöglicht, wobei das Endprodukt sowohl flächenelastische Eigenschaften zur optimalen Anpassung an die Körperkontur des Benutzers, als auch volumenelastische Eigenschaften zur Aufnahme großer Flüssigkeitsmengen aufweist.

Diese Aufgabe wird erfindungsgemäß dadurch gelöst, daß durch die Einbringung von Superabsorber und einer oder mehrerer elastischer Zwischenlagen, bestehend aus Einzelfäden, -strängen oder Bändern zwischen zwei äußere Lagen eines so hergestellten Laminats dieses dahingehend im Wesentlichen quer zur Herstellungsrichtung dehnbar gemacht und im entspannten Zustand gerafft wird, daß die elastischen Zwischenlagen einzelne Sektionen von Superabsorber in der Art einer Steppdecke einschließen und der Ausdehnung des Laminats bei der Flüssigkeitsaufnahme senkrecht zur und in der Produktionsebene Raum schaffen. Für die Verwendung im Rahmen moderner ultradünner und elastischer Hygieneartikel ist es ferner von Bedeutung, daß dieses so erzeugte Laminat vollständig elastisch und dehnbar ist und sich sowohl im trockenen, als auch im feuchten Zustand perfekt der Körperkontur anpaßt.

Durch die so erzielte Raffung und Textur der hautseitigen Oberfläche des Laminats, bzw derjenigen Seite, von der die aufzunehmende Flüssigkeit das Laminat trifft und vorteilhafterweise in Verbindung mit einer entsprechend hydrophilen und transportfähigen äußeren Schicht ist eine ausgezeichnete Flüssigkeitsleitung sowohl in trockenem, als auch in feuchtem Zustand gewährleistet. Auch ist ein so hergestelltes Laminat selbst in gequollenem Zustand im Bereich der Verleimung der elastischen Lagen jederzeit senkrecht zu seiner Produktionsebene durchlässig, ohne durch die Quellung des Superabsorbers behindert zu werden, so daß die Transportleistung auch auf der hautabgewandten Seite durch die Textur der Oberfläche und die Wahl der entsprechenden äußeren Schicht gewährleistet ist und die hautseitige äußere Schicht hinsichtlich Rücknässung/Hautfeuchte optimiert werden kann.

Erfindungsgemäß wird so vorgegangen, wie in den Ansprüchen vollständig definiert, daß zunächst das vordere Ende der ersten äußeren textilen Schicht 20 auf den Endabschnitt eines langgestreckten stab- oder rohrförmigen Formkerns 5 aufgebracht und unter Erteilung einer Vorzugsbewegung um den Formkern herum in eine schlauchartig geschlossene Form gefaltet wird.

Im weiteren Verlauf der Vorzugsbewegung auf dem Formkern wird durch Verjüngung des Querschnitts des Formkerns der Umfang dieses Schlauchs dahingehend verkürzt, daß überschüssiges Material durch außerhalb des Schlauches liegende Führungsschienen oder -stäbe 8 in geeignete Aussparungen des Formkerns 5 geführt wird, anschließend durch zwei oder mehrere, den Formkern ringförmig umgebende und paarweise gegenläufige, angetriebene Zuführvorrichtungen 11,14 von in Umfangsrichtung beabstandeten Einzelführungen 17 Gruppen von elastischen Fäden oder Strängen 50,53 lose abgezogen und ohne oder nur mit reibungsbedingter geringer Vorspannung um die erste äußeren Materialschicht im Verlauf des Vorzugs auf dem Formkern aufgelegt. Auf das die den Schlauch der ersten äußeren Materialbahn kreuzförmig-symmetrisch umgebenden Flächenmuster 32 der elastischen Fäden oder Stränge wird ein Haftkleber 29 fadenförmig aufgebracht, welcher die im Wesentlichen frei liegenden elastischen Fäden benetzt und umschlingt. Im weiterem Verlauf der Vorschubbewegung wird durch Vergrößerung der Querschnitts des Formkerns der Schlauch der ersten äußeren Materialbahn gestrafft und so mit den elastischen Fäden in Kontakt gebracht und letztlich in eine flache Form gebracht. Beidseits dieses flachen Schlauches werden mittels eines Paars von Vorzugsoder Andruckwalzen 38 zwei Einzelbahnen der zweiten äußeren Materialschicht 32 auf die freie Oberfläche der ersten äußeren Materialbahn 20 und der mit dem Haftkleber 29 versehenen elastischen Fäden 17 verbunden. Während dieser Verbindung werden beidseits des Schlauchs der ersten äußeren Materialbahn zwischen dieser und der zugeführten zweiten äußeren Materialbahn einzelne Spuren von Superabsorber 47 eingebracht. Abschließend wird der so erzeugte Schlauch längs in Einzelbahnen aufgetrennt und diese traversierend auf Einzelrollen gewickelt oder in Kisten abgelegt.

Durch die Einfaltung des Schlauches der ersten Materialbahn und Erzeugung der Umfangselastizität des Schlauches durch Aufspreizung der ersten äußeren Materialbahn unter Ausdehnung des elastischen Fadenmusters 32 ergibt sich gegenüber vergleichbaren Wickelverfahren für elastische Schläuche auf der Basis von vorgespannten Fäden oder Bändern (z.B. WO 03041627, DE 102004026070) eine erhebliche Reduzierung der Komplexität durch Eliminierung des Antriebs der Einzelführungen der Fäden und eine proportional zur Querdehnbarkeit des Laminats erfolgende Erhöhung der Produktionsmenge, da erfahrungsgemäß die Produktionsgeschwindigkeit durch die Grenzdrehzahl der ringförmigen Zuführvorrichtungen begrenzt wird. Ferner wird die Breite des Auftragssystem für den Haftkleber 29 in selbem Maße reduziert.

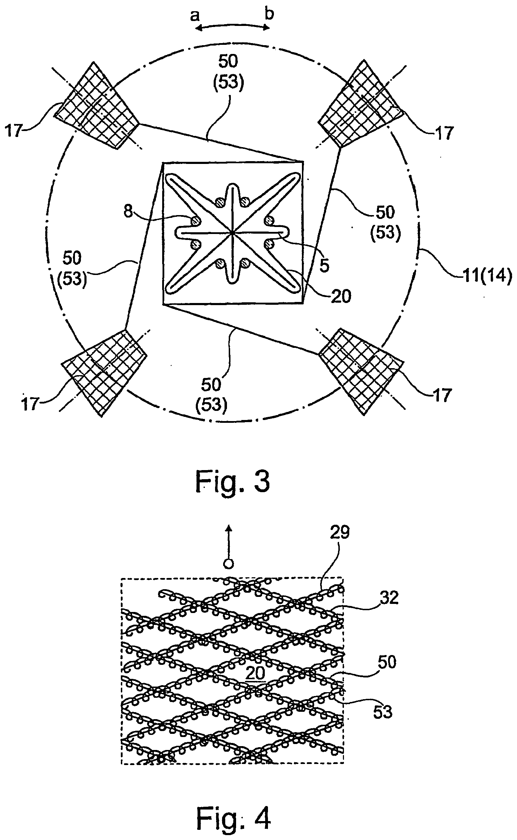

Da nach Aufbringung der elastischen Fäden oder Bänder auf den Schlauch der ersten äußeren Materialbahn diesen nur punktuell berühren und ansonsten frei liegen, wird ein spiral- oder meanderförmig aufgebrachter Haftkleber durch das Flächenmuster der elastischen Fäden gezwungen, bevorzugt diese zu umschlingen und nach Aufspreizung des Schlauches der ersten äußeren Materialbahn sich im Wesentlichen um die Fäden orientiert, wodurch ein dem Flächenmuster 32 entsprechendes Leimbild (Fig. 4) erzeugt wird welches aufgrund der Umschlingung der elastischen Fäden diese sowohl mit der ersten, als auch mit der zweiten äußeren Materialbahn im weiteren Verlauf der Vorzugsbewegung auf dem Formkern haftend verbindet.

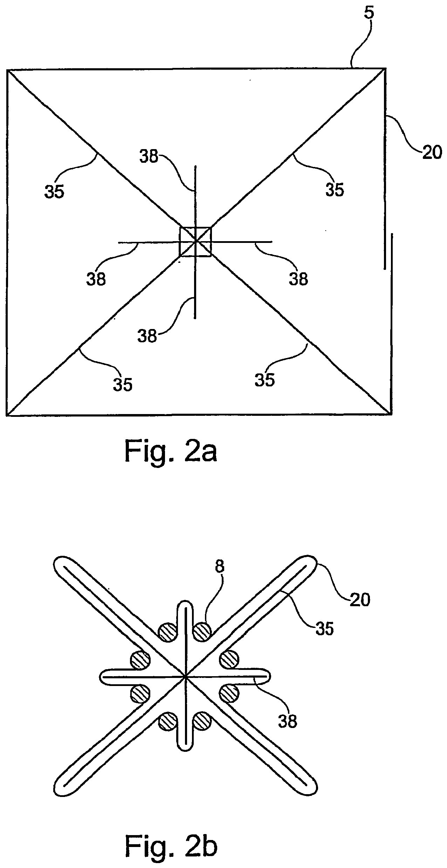

Es hat sich als zweckmäßig erwiesen, den Formkern 5 entsprechend Bild 2a aus Führungsstreben 35, welche die Materialbahn 20 führen, und dazwischen angeordnete Speicherstreben 38 auszubilden (Fig. 2a), in welche während der Verjüngung des Formkerns durch entsprechende Führungsschienen 8 überschüssiges Material des Schlauches der Materialbahn 20 eingedrückt wird (Fig. 2b).

Einmal in die Speicherstreben eingedrückt, kann die erste äußere Materialschicht leicht durch Unterdruck, Einbringung von Pressluft oder durch elektrostatische Aufladung in einer an die Führungs- und Speicherstreben des Formkerns eng anliegenden Position gehalten werden und reibungsarm über den Formkern gezogen werden.

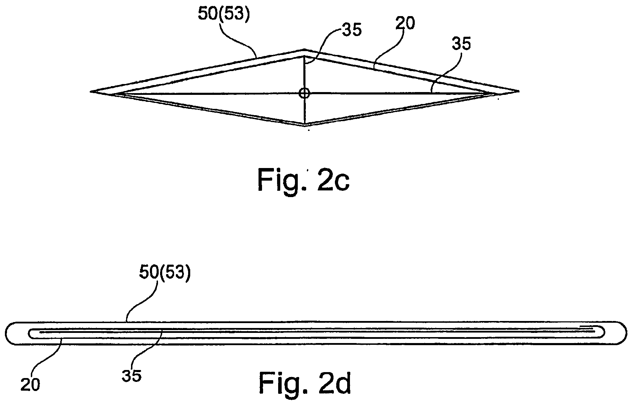

Die Aufspreitzung des Formkerns nach Aufbringung der elastischen Zwischenlagen und des Haftklebers wird vorteilhafterweise dadurch gelöst, daß paarweise gegenüberliegende Führungsstreben im weiteren Verlauf des Formkerns in ihrer Ausdehnungsrichtung quer zur Längsachse des Formkerns sukzessive verbreitert, ein weiteres Paar Führungsstreben in selbem Maße in seiner Breite verringert wird, so daß der Schlauch der ersten äußeren Schicht kontinuierlich aus den Speicherstreben gezogen und über die Spitzen der Führungsstreben straff gezogen und so mit den haftverleimten elastischen Lagen in flächigen Kontakt gebracht wird (Fig.2c), letztlich im weiteren Verlauf der Vorzugsbewegung durch weitere synchrone paarweise Verbreiterung, respektive Breitenreduzierung der Führungsstreben bei im Wesentlichen gleicher Umfangslänge in einen flachen Zustand gebracht wird (Fig. 2d).

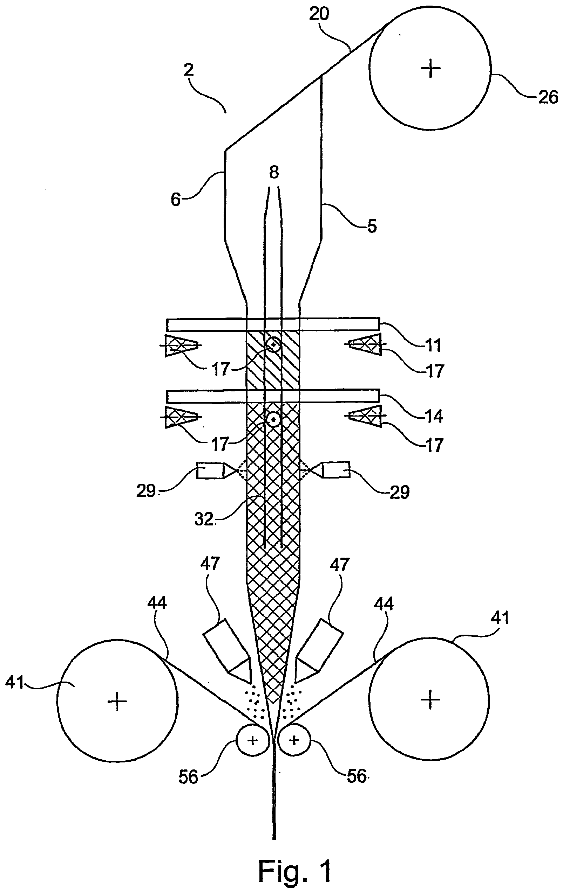

Vorteilhafterweise wird der Formkern 5 dieses Prozesses mit seiner Längsrichtung dergestalt senkrecht angeordnet, daß die Vorzugsrollen 56 sich am unteren Ende des Formkerns 5 befinden (Fig.1).

Die Zufuhr des Superabsorbers 47 erfolgt zweckmäßigerweise in individuellen volumetrisch oder gewichtsdosierten kontinuierlichen Einzelbahnen oder -spuren, welche entweder intermittierend durch pulsierende Druckluft oder Kolben in die leimfreien Bereiche des Flächenmusters eingetragen oder intermittierend dem Flächenmuster 32 folgend quer zur Fließrichtung des Schlauchs der ersten äußeren Materialbahn ausgelenkt werden. Für einfache Anwendung kann es hinreichend sein, diese Einzelspuren kontinuierlich einrieseln zu lassen, wobei zweckmäßigerweise pro Kassette des Flächenmusters zwei Einzelbahnen zugeführt werden.

Es kann sinnvoll und zweckmäßig sein, den Schlauch der ersten äußeren Materialbahn 20 vor der Zufuhr in die Zugrollen 56 in Querrichtung durch Verringerung der Breite der Führungsstreben zu entspannen, so daß diese bei der Zuführung der zweiten äußeren Bahn 44 eine leichte Welligkeit aufweist, wodurch sich im Bereich der nicht verleimten Zonen des Flächenmusters 26 kleine Taschen bilden, die die Ablage des Superabsorbers 33 erleichtern.

In Weiterentwicklung der Erfindung kann durch geeignete Kontrolle dieser Welligkeit das Verhältnis der Materialbreiten der ersten und zweiten äußeren Materialbahnen zueinander dahingehend gesteuert werden, daß das so erzeugte Laminat auf der Seite der ersten äußeren Schicht eine höhere Welligkeit gegenüber der Seite der zweiten äußeren Schicht aufweist. Hierdurch ist bei gleichem Quellvolumen der einzelnen Kassetten eine Minimierung des jeweils teueren Materials der äußeren Schichten 20&44 möglich.

### BESCHREIBUNG DER ZEICHNUNGEN

In der folgenden Beschreibung ist die vorliegende Erfindung in Verbindung mit den Zeichnungen näher erläutert, welche schematisch die Durchführung des erfindungsgemäßen Verfahrens bei der Herstellung von flächenelastischem saugfähigem Bahnmaterial veranschaulichen. Es zeigen
- Fig.1: Stark schematisierte Darstellung der Seitenansicht der wesentlichen Funktionselemente einer zur Durchführung des erfindungsgemäßen Verfahrens verwendbaren Vorrichtung.
- Fig. 2a: Aufbau des Formkerns aus Führungs- und Speicherstreben.
- Fig. 2b: Aufbau des Formkerns aus Führungs- und Speicherstreben und Führungsschienen im Bereich der Verjüngung des Formkerns
- Fig. 2c: Aufbau des Formkerns im Bereich der Aufspreizung des Formkerns
- Fig. 2d: Aufbau des Formkerns nach vollendeter Aufspreizung
- Fig. 3: Die Anordnung von Einzelführungen in einer der in der Vorrichtung gemäß den Figuren 1 und 2 zur Aufbringung von Gruppen von ungespannten oder leicht gespannten Fäden oder Strängen vorgesehene Zuführeinrichtungen.
- Fig. 4: Das bei der Verwendung von zwei gegensinnig angetriebenen Zuführeinrichtungen gemäß Fig. 3 erhaltene Flächenmuster der elastischen Fäden oder Stränge und des aufgebrachten Haftklebers in einem in der erfindungsgemäßen Weise hergestellten Bahnmaterial.
- Fig. 5a: das bei der Verwendung von zwei gegensinnig angetriebenen Zuführeinrichtungen gemäß Fig. 3 erhaltene Flächenmuster der elastischen Fäden oder Stränge und des punktuell zugeführten pulverförmigen Füllmaterials in einem in der erfindungsgemäßen Weise hergestellten Bahnmaterial.
- Fig. 5b: das bei der Verwendung von zwei gegensinnig angetriebenen Zuführeinrichtungen gemäß Fig. 3 erhaltene Flächenmuster der elastischen Fäden oder Stränge und des kontinuierlich in diskreten Einzelbahnen zugeführten pulverförmigen Füllmaterials in einem in der erfindungsgemäßen Weise hergestellten Bahnmaterial.
- Fig. 5c: das bei der Verwendung von zwei gegensinnig angetriebenen Zuführeinrichtungen gemäß Fig. 3 erhaltene Flächenmuster der elastischen Fäden oder Stränge und des kontinuierlich in diskreten Einzelbahnen zugeführten, quer zur Produktionsrichtung intermittierend ausgelenkten pulverförmigen Füllmaterials in einem in der erfindungsgemäßen Weise hergestellten Bahnmaterial.
- Fig.6: Schematische Darstellung des erfindungsgemäß hergestellten Laminats bei Entlastung des Schlauches der ersten Materialbahn und der eingelagerten elastischen Zwischenlagen quer zu deren Produktionsrichtung vor Einbringung des Superabsorbers und der Verbindung mit der zweiten äußeren Schicht.
- Fig 7a: Schematische Darstellung eines Ausschnitts des Querschnitts des Formkerns bei Verwendung von zwei Speicherstreben zwischen jeweils zwei Führungsstreben und der Kontrolle des sektionsweisen Einzugs der ersten äußeren Schicht durch Positionierung der Führungsschienen.
- Fig. 7b: Flächenmuster der elastischen Fäden des nach Fig. 7a hergestellten elastischen Bahnmaterials in ebenen, entspannten Zustand.

Die in Fig 1 vereinfacht und stark schematisiert dargestellte Vorrichtung 2 zur Durchführung des erfinderischen Verfahrens besteht aus einem langgestreckten Formkern 5 mit in diesem Fall quadratischem Querschnitt auf, welcher durch zwei in Längsrichtung voneinander beabstandeten Zuführeinrichtungen 11,14 umgeben ist. Diese Zuführeinrichtungen sind im Sinne der durch die Pfeile a und b veranschaulichten Richtungen gegensinnig drehangetrieben. Auf diesen ringförmigen Zuführeinrichtungen sind jeweils in Umfangsrichtung verteilt voneinander beabstandete Einzelführungen 17 für elastische Fäden, Bänder oder Stränge angeordnet, über welche die elastischen Elemente in axialer Richtung zu ihrer Aufwicklung abgezogen und in Richtung zum Formkern 5 geführt werden.

Von einer drehbar gelagerten und angetriebenen Materialrolle 26 wird eine erste textile Schicht 20 vorzugsweise bahnspannungsgesteuert gefördert, über eine Formschulter 6 in einen Schlauch rechteckigen Querschnitts gefaltet und auf das vordere Ende des in diesem Beispiel durch mehrere Streben 35,38 gebildeten Formkerns 5 aufgelegt (Fig 2a). Vorteilhafterweise wird hier die Überlappung der ersten textilen Schicht 20 bei der Bildung des Schlauches durch Aufbringung eines Haftklebers, Verschweißen oder mechanische Verhaftung fixiert.

Dieser so gebildete Schlauch wird anschließend über den sich in seinem Querschnitt verjüngenden Formkern derart geführt, das dieser den Formkern lediglich auf den Enden der sich in ihrer Breite verkürzenden Führungsstreben 35 geführt und dazwischenliegendes Material des Schlauches mittels Führungsschienen oder -drähten 8 in durch zwischen den Führungsstreben angeordneten Speicherstreben 38 geformte Freiräume geführt wird (Fig 2b).

Dieser so in seinem Umfang eingekürzte Schlauch wird anschließend durch zwei gegenläufig drehende Zuführeinrichtungen 11,14 geführt. In den Zuführeinrichtungen werden von jeweils in Umfangsrichtung beabstandeten Einzelführungen 17 stationärer Spulen oder Rollen einzelne elastische Fäden, Bänder oder Stränge 50,53 abgezogen und auf den Schlauch der ersten äußeren Materialschicht 20 aufgelegt. Durch die Überlagerung der Drehbewegungen der beiden Zuführeinrichtungen 11,14 mit dem Vorzug der ersten Materialschicht 20 ergibt sich ein gegenläufig-diagonales Muster der elastischen Elemente 50,53, welches mit der ersten Materialschicht lediglich auf den Spitzen der Führungsschienen 35 des Formkerns 5 in Kontakt ist, ansonsten aber frei liegt, Fig 3.

Im weiteren Verlauf des Vorzugs auf dem Formkern wird auf die elastischen Fäden 50,53 Haftkleber bevorzugt als ein Vorhang von Spiral- oder Mäanderfäden aufgetragen, welcher bei Aufschlag auf das freiliegende gegenläufig-diagonale Muster der elastischen Fäden 50,53 von diesen umgelenkt und bevorzugt die elastischen Fäden, speziell aber die Kreuzungspunkte der Fäden 50 und 53 zueinander benetzt und umfängt, Fig 4.

Im weiteren Verlauf des Vorzugs auf dem Formkern 5 wird dieser in seinem Querschnitt derart gespreizt, daß jeweils zwei gegenüberliegende Führungsstreben in ihrer Breite dahingehend erweitert und ein komplementäres Paar von Führungsstreben in seiner Breite dahingehend verringert wird, das zunächst das in den Speicherstreben 38 des Formkerns 5 verbliebene Material der ersten Schicht 20 kontinuierlich gelöst wird und der Schlauch der ersten Materialschicht 20 über die Enden der Führungsschienen 35 straff gespannt wird (Fig 2c). Hierdurch wird der Schlauch der ersten Materialbahn 20 flächig in Kontakt mit dem Muster der elastischen Elemente 17 und des auf diese aufgebrachten Haftklebers gebracht. Der Prozeß der paarweisen Erweiterung der Führungsstreben 35 und der einhergehenden Verkleinerung komplementärer Führungsstreben wird im Verlauf des weiteren Vorzugs des Schlauchs der ersten Materialbahn 20 über den Formkern 5 dahingehend fortgeführt, das letztlich der Schlauch der ersten Materialbahn 20 nur über die Enden von zwei Führungsstreben 35 des Formkerns 5 geführt wird und damit praktisch eben ist.

In einem weiteren Schritt im Verlauf des Vorzugs wird dieser so geformte und in eine flache Form gebrachte Schlauch in eine Vorrichtung von zwei angetriebenen gegenläufigen Vorzugselementen, hier zwei Rollen oder Walzen 56 eingeführt, welche letztlich den Vorzug für den Abzug der ersten äußeren Schicht 20 von der Materialrolle 26 und den Transport aller Materialien über den Formkern liefert.

Über die Vorzugswalzen 56 wird beiderseits der Materialbahn 20 von zwei von einander beabstandeten, drehend angetriebenen Vorrichtungen von Materialrollen 41 je eine Einzelbahn der zweiten äußeren Schicht 44 vorzugsweise bahnspannungskontrolliert abgezogen und zugeführt und mit der ersten Materialschicht 20 und dem elastischen Flächenmuster 32 flächig über den Haftkleber 29 verbunden.

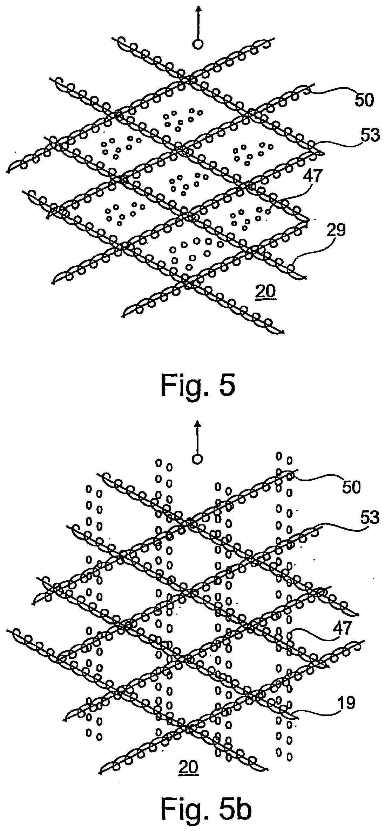

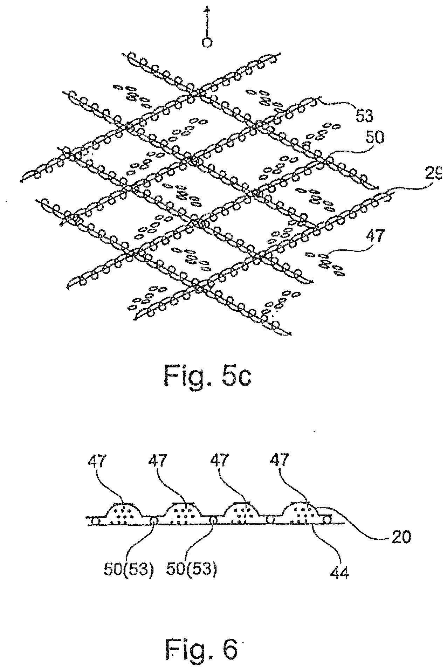

In die Vereinigung von abgeflachtem Schlauch der ersten Materialbahn 20 mit den zugeführten ebenen Bahnen der zweiten Materialbahn 44 durch die Vorzugseinheit 56 wird beidseitig des Schlauches senkrecht ein Superabsorber 47 derart eingebracht, das entsprechend auf das Flächenmuster 32 abgestimmte, einzeln volumetrisch oder grammetrisch auf die Vorzugsgeschwindigkeit des Schlauches der ersten Materialbahn 20 abgestimmte Einzelbahnen oder -spuren kontinuierlich eingerieselt werden, wodurch sich ein Produktmuster gemäß Fig 5b ergibt, alternativ diese Spuren intermittierend oder oszillierend quer zur Fließrichtung entsprechend dem Flächenmuster 32 mechanisch oder pneumatisch abgelenkt (Produktmuster gemäß Fig 5c) oder diese Spuren entweder durch pulsierende oder intermittierende Druckluft, mechanische Kolben oder mechanische Auslenkung der Fördereinrichtung quer zu Fließrichtung des Schlauches der ersten Materialbahn 20 auf die Zentren der durch das Flächenmuster 32 gebildeten haftkleberfreien Bereiche dieses Schlauches gelenkt werden (Produktmuster Fig 5a).

Nach Abbinden des Haftkleber, vorteilhafterweise durch den Andruck der Vorzugsrollen 56 erreicht und nach der Verbindung der beiden äußeren Schichten 20 und 44 untereinander mit den dazwischen eingelagerten elastischen Elementen 50,53 unter Einschluß des eingetragenen Superabsorbers wird im Verlauf der weiteren Vorzugsbewegung dieser so geformte mehrlagige Schlauch längs in einzelne Bahnen aufgetrennt, welche wahlweise traversierend auf Rollen aufgewickelt oder in Kisten abgelegt oder aufgeschossen werden können.

In Weiterentwicklung des hier beschriebenen erfinderischen Verfahrens kann die Breite des flachen Schlauches aus erster textiler Schicht 20 und Flächenmuster der elastischen Elemente 32 nach Aufspreizen in den flachen Zustand Fig 2d durch Verringerung der Breite der Führungsschienen 35 derart entspannt und gerafft werden, daß sich nach Verbindung mit der ansonsten im Wesentlichen flachen zweiten äußeren Schicht 44 ein elastisches, saugfähiges Bahnmaterial ergibt, welches auf der durch die erste textile Schicht gebildeten Außenseite eine höhere Raffung aufweist als auf der durch die zweite äußere Schicht geformten Seite, Fig 6.

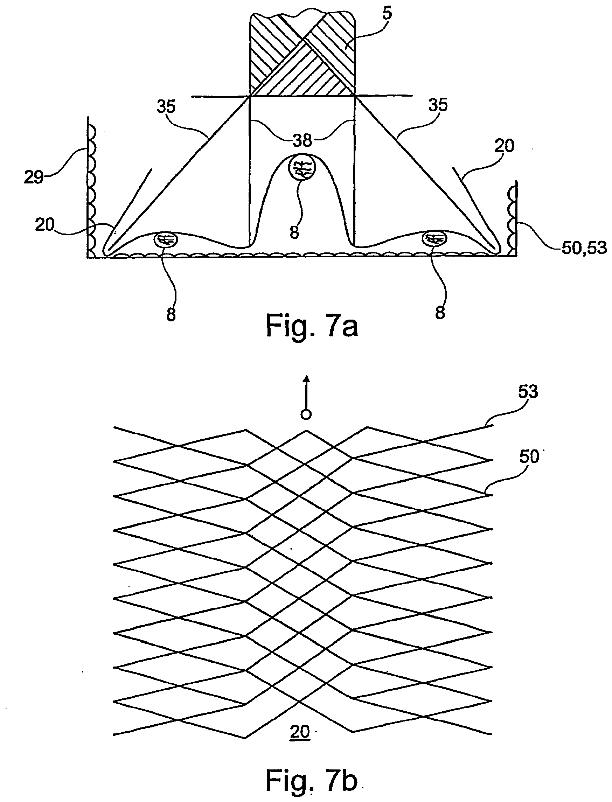

In einer weiteren Entwicklung des erfinderischen Verfahrens kann es wünschenswert und zweckmäßig sein, das so hergestellte ebene Bahnmaterial quer zu seiner Herstellungsrichtung mit Sektionen unterschiedlicher Raffung der äußeren Lagen und somit unterschiedlichen Quellvolumens auszustatten, was durch Steuerung der Superabsorberverteilung quer zur Herstellungsrichtung des Bahnmaterials zu einer vorteilhaften Querverteilung der Absorptionsfähigkeit führt. In Fig 7a ist beispielhaft dargestellt, wie bei in diesem Fall mit zwei Speicherstreben 38 pro Führungsstrebenpaar 38 durch geeignete Anordnung der Führungsschienen 8 in den jeweils ausgeformten Speichersektionen des Formkerns 5 in Querrichtung zur Vorzugsrichtung unterschiedliche Bahnbreiten des durch die Verjüngung des Formkerns 5 überschüssigen Bahnmaterials der ersten äußeren Schicht 20 eingefügt werden. Werden nun im weiteren Verlauf der Vorschubsbewegung und Aufspreitzung des Formkerns 5 zunächst durch Verbreiterung der Speicherstreben 38 auf deren Spitzen die Materialbahn 20 mit dem Haftkleber 29 der elastischen Lagen 50,53 in Kontakt gebracht und anschließend die Aufspreizung des Formkerns 5 im Sinne der Fig. 2b-d fortgeführt und die Kombination mit dem Superabsorber und der zweiten äußeren Schicht 44 vollendet, so entsteht letztlich ein längs aufgetrenntes Bahnmaterial, welches in entspanntem, ebenem Zustand eine in Querrichtung streifenweise Variation der Raffung der äußeren Schichten und damit letztlich der Absorptionsfähigkeit aufweist, Fig 7b.

Es kann zweckmäßig sein, die Funktion des Eindrückens der ersten textilen Schicht in die Freiräume der Speicherstreben anstelle der Führungsschienen 8 durch Unterdruck aus dem Führungskern, durch statische Aufladung der ersten Materialbahn 20 gegenüber den Streben 35,38 des Formkerns oder durch Aufbringung von Druckluft auf die Außenseite des Schlauches der ersten Materialschicht 20 zu erreichen.

Es kann ferner sinnvoll und zweckmäßig sein, die Funktionen der jeweils 4 Führungs- und Speicherstreben aus Fig 2a und 2b durch eine entsprechend höhere Zahl an Streben zu erreichen, um speziell bei großen Bahnbreiten der ersten äußeren Schicht 20 oder bei hohem Speicherbedarf bei der Verjüngung der Formkerns 5 eine gute Materialführung zu erreichen.

Auch wenn die Isolierung einzelner Sektionen von Superabsorbern in leimfreien Bereichen eines ansonsten untereinander verleimten Bahnmaterial aus zwei äußeren Schichten 20,44 und eingelagerten elastischen Lagen 50,53 für die Quellwirkung und Volumenvergrößerung des Superabsorbers 47 bei der verwendungsgemäßen Flüssigkeitsaufnahme zweckmäßig ist, kann es sinnvoll sein, die zweite äußere Schicht 44 vor Kombination mit der ersten äußeren Schicht 20 flächig oder in Längsstreifen mit einer zusätzlichen leichten Schicht Haftkleber zu benetzen, um den Superabsorber möglichst flächig zu fixieren, dadurch einem möglichen Verklumpen dieses Materials vor Beginn der Flüssigkeitsaufnahme vorzubeugen und die Haptik des so hergestellten Bahnmaterials zu verbessern.

### BEZEICHNUNGEN DER ELEMENTE DER ZEICHNUNGEN

- 2: Gesamtvorrichtung
- 5: Formkern
- 6: Formschulter der ersten textilen Schicht
- 8: Führungen erste textile Schicht
- 11: Erste Zuführeinrichtung
- 14: Zweite Zuführeinrichtung
- 17: Einzelführung elastische Fäden
- 20: Erste textile Schicht
- 23: Formschulter
- 26: Materialrolle der ersten textilen Schicht
- 29: Haftkleber
- 32: Flächenmuster der elastischen Fäden
- 35: Führungsstreben des Formkerns
- 38: Speicherstreben des Formkerns
- 41: Materialrolle der zweiten textilen Schicht
- 44: Zweite textile Schicht
- 47: Superabsorber
- 50: Einzelfaden der ersten Zwischenlage

- 53: Einzelfaden der zweiten Zwischenlage
- 56: Zugvorrichtung

## Patentansprüche

1. Verfahren zur Herstellung eines biegeweichen, absorbierenden Bahnmaterials, bestehend aus zwei äußeren textilen Schichten (20, 44), zwischen denen zwei unter Vorspannung stehende elastische Zwischenlagen und Sektionen von superabsorbierendem Granulat eingebracht sind, hergestellt derart, dass eine textile äußere Schicht über einen langgestreckten Formkern (5) zu einem Schlauch gefaltet, dieser durch Verjüngung des Querschnitts des Formkerns (5) verkleinert und mit Gruppen ungespannter elastischer Fäden oder Bänder gegenläufig im Sinne einer Schlaucharmierung umwickelt, auf diese Gruppen elastischer Elemente Haftkleber (29) aufgebracht und diese durch Spreizung über den Formkern (5) unter Spannung mit dem Schlauch der ersten äußeren Schicht (20) in Kontakt, beide zusammen im weiteren Verlauf der Vorschubbewegung des Schlauches auf dem Formkern (5) in eine flache Form gebracht und unter intermittierender oder kontinuierlicher Zufuhr von Einzelspuren von Superabsorber (47) mit zwei Bahnen einer zweiten äußeren Schicht (44) verbunden und längs zu querelastischen Einzelbanen aufgeschnitten wird.

2. Verfahren nach Anspruch 1, **gekennzeichnet dadurch, dass** mindestens eine der äußere Lagen wasser-undurchlässig ist, in Kombination mit dem Haftkleber (29) aber durchlässig gemacht wird.

3. Verfahren nach Anspruch 1-2, **gekennzeichnet dadurch, dass** mindestens eine der äußere Lagen wasserundurchlässig ist, durch mechanischen Druck oder Perforation während der Verbindung der beiden äußeren Lagen punktuell durchlässig gemacht wird.

4. Verfahren nach Anspruch 1-3, **dadurch gekennzeichnet**, das die erste Materialschicht unter Ausnutzung der Vorspannung der aufgebrachten elastischen Lagen vor der Verbindung mit der zweiten äußeren Lage derart in seiner Querrichtung entspannt und verkürzt wird, das in Kombination mit der zweiten äußeren Materialbahn das so entstehende Bahnmaterial auf der durch die erste äußere Schicht (20) geformten Bahnseite eine erhöhte Raffung aufweist.

5. Verfahren nach Anspruch 1-4, **dadurch gekennzeichnet, dass** auf die dem Superabsorber (47) zugewandten Seite der zweiten äußeren Schicht (44) ein zusätzlicher Haftkleber zur flächigen Fixierung des Superabsorbers (47) im trockenen Zustand aufgetragen wird.

## Claims

1. Process for the production of a flexurally yielding, absorbent web material, consisting of two exterior textile layers (20, 44), between which are incorporated two pretensioned elastic interplies and sections of superabsorbent granulate, produced such that a textile exterior layer is folded over an elongate shaping core (5) to give a flexible tube, the size of this flexible tube is reduced by narrowing of the cross section of the shaping core (5), and groups of untensioned elastic threads or bands are wound in contrarotating manner around the said tube in the manner of reinforcement of the flexible tube, pressure-sensitive adhesive (29) is applied to these groups of elastic elements and, by spreading over the shaping core (5) under tension, these are brought into contact with the flexible tube of the first exterior layer (20), both together are converted, in the further course of the forward feed motion of the flexible tube on the shaping core (5), to a flat shape, and by intermittent or continuous supply of individual tracks of superabsorbent (47), are bonded to two lengths of a second exterior layer (44), and are cut open longitudinally to give separate transversely elastic webs.

2. Process according to Claim 1, **characterized in that** at least one of the exterior plies is impermeable to water, but in combination with the pressure-sensitive adhesive (29) is rendered permeable.

3. Process according to Claim 1-2, **characterized in that** at least one of the exterior plies is impermeable to water, and by virtue of mechanical pressure or perforation during the bonding of the two exterior plies is rendered punctiformly permeable.

4. Process according to Claim 1-3, **characterized in that**, with utilization of the pretensioning of the applied elastic plies, prior to the bonding to the second exterior ply, the first layer of material is relaxed and shortened in its transverse direction in a manner such that, in combination with the second exterior web of material, the resultant web material has increased shirring on the web side formed by the first exterior layer (20).

5. Process according to Claim 1-4, **characterized in that** the superabsorbent-(47)-facing side of the second exterior layer (44) receives an additional pressure-sensitive adhesive applied to it in the dry state for areal fixing of the superabsorbent (47).

## Revendications

1. Procédé de fabrication d'un matériau en bande absorbant et souple, composé de deux couches textiles extérieures (20, 44) entre lesquelles deux couches intermédiaires et sections élastiques sous précontrainte d'un granulat superabsorbant sont introduites, qui est fabriqué de telle sorte qu'une couche extérieure textile est pliée sur un noyau de mise en forme allongé (5) pour former un tuyau, celui-ci est rendu plus petit par le rétrécissement de la section transversale du noyau de mise en forme (5), et est enrobé de groupes de fils ou de bandes élastiques sans tension en sens opposé à la manière d'une armure de tuyau, un adhésif (29) est appliqué sur ces groupes d'éléments élastiques, et ceux-ci sont mis en contact avec le tuyau de la première couche extérieure (20) par écartement sur le noyau de mise en forme (5) sous tension, les deux ensemble sont mis dans une forme plate dans la suite du mouvement d'avance du tuyau sur le noyau de mise en forme (5), et sont reliés à deux bandes d'une deuxième couche extérieure (44) par un apport intermittent ou continu de traces individuelles d'un matériau superabsorbant (47), et sont découpés en longueur pour former des bandes individuelles extensibles en largeur.

2. Procédé selon la revendication 1, **caractérisé en ce qu'**au moins l'une des couches extérieures est imperméable à l'eau mais est rendue perméable en combinaison avec l'adhésif (29).

3. Procédé selon les revendications 1 à 2, **caractérisé en ce qu'**au moins l'une des couches extérieures est imperméable à l'eau et rendue ponctuellement perméable par pression mécanique ou perforation pendant l'assemblage des deux couches extérieures.

4. Procédé selon les revendications 1 à 3, **caractérisé en ce que** la première couche de matériau est détendue et raccourcie dans sa direction transversale en exploitant la précontrainte des couches élastiques appliquées avant l'assemblage avec la deuxième couche extérieure de telle sorte qu'en combinaison avec la deuxième bande de matériau extérieure, le matériau de bande ainsi créé présente un plissage accru sur la face de bande formée par la première couche extérieure (20).

5. Procédé selon les revendications 1 à 4, **caractérisé en ce que** sur le côté, tourné vers le matériau superabsorbant (47), de la deuxième couche extérieure (44), un adhésif supplémentaire est appliqué à l'état sec pour la fixation en nappe du matériau superabsorbant (47).
